# EUROPÄISCHE PATENTSCHRIFT

(11) **EP 1 514 700 B1**
(45) Veröffentlichungstag und Bekanntmachung des Hinweises auf die Patenterteilung: **14.01.2009**
(21) Anmeldenummer: 04001268.4
(22) Anmeldetag: 22.01.2004
(51) Int. Cl.: B43K 19/16, B43K 19/00, A45D 40/20

(54) **Holzgefasster Stift für Schreib-, Mal-, Zeichen- und Kosmetikzwecke**
Pencil encased in wood for writing, colouring, drawing and cosmetic purposes
Crayon à gaine de bois pour écrire, colorer, dessiner ainsi que pour un usage cosmétique

(30) Priorität: 12.09.2003 DE 20314274 U
(43) Veröffentlichungstag der Anmeldung: 16.03.2005
(73) Patentinhaber: Faber-Castell AG, 90546 Stein (DE)
(72) Erfinder: Lugert, Gerhard, Dr., 90431 Nürnberg (DE); Beck, Udo, 90461 Nürnberg (DE); Oetter, Walter, 90546 Stein (DE)
(74) Vertreter: Mörtel & Höfner

(56) Entgegenhaltungen:
- EP-A- 0 181 528
- WO-A-00/68023
- DE-A1- 2 610 247
- DE-A1- 2 938 292

## Beschreibung

Die Erfindung betrifft ein Verfahren zur Herstellung eines holzgefassten Stiftes für Schreib-, Mal-, Zeichen- und Kosmetikzwecke. Solche Stifte weisen einen Schutzmantel auf, in dem zentral eine Mine gelagert ist. Unter dem Begriff Stifte sollen aber auch stiftförmige Halter für Minen verstanden werden. Holzgefasste Stifte weisen in der Regel eine durch Lacküberzug gebildete glatte Oberfläche auf. Das Festhalten des Stiftes ist daher mit einem größeren Kraftaufwand verbunden, was bei längerem Gebrauch ermüdet.

Aus EP 1 177 108 ist ein Stift der eingangs benannten Art bekannt, bei dem die zur Handhabung dienende Oberfläche Griffflächen oder Griffnoppen bildende erhabene Strukturen aus einem Kunststoffmaterial aufweist. Die Griffigkeit des Stiftes wird schon allein durch die Anwesenheit der erhabenen Strukturen erhöht. Eine weitere Verbesserung der Griffigkeit bzw. taktilen Eigenschaften kann durch Auswahl eines entsprechenden Kunststoffmaterials erreicht werden. Die erhabenen Strukturen sind bei dem bekannten Stift in Form einer zunächst fließfähigen Kunststoffmasse auf die Stiftoberfläche aufgebracht, wobei diese sich anschließend verfestigt oder verfestigbar ist. Der Hauptbestandteil der Kunststoffmasse ist eine wässrige, wasserfeste erhärtende Polymerdispersion oder eine Mischung solcher Dispersionen.

Aus JP-A-09/039467 sind Stifthülsen aus Kunststoff oder Metall bekannt, auf deren Oberfläche erhabene Strukturen auf die oben erwähnte Art und Weise aufgebracht sind. Bei der zunächst fließfähigen Kunststoffzubereitung handelt es sich um eine Mischung aus einem Polyurethanharz, Polyol und zumindest einem Härter für das Polyurethanharz. Die im Siebdruck auf die Stifthülse aufgebrachten Strukturen werden bei Temperaturen ausgehärtet, die oberhalb von 80°C liegen. Solche Temperaturen sind für lackierte Holzstifte, die außerdem oft empfindliche Minen enthalten, schädlich, so dass die aus der genannten Druckschrift bekannten Noppen nicht für holzgefasste Stifte anwendbar sind.

Aus WO 00/68023 A geht ein holzgefasster Stift für Schreib-, Mal-, Zeichen- und Kosmetikzwecke hervor, dessen der Handhabung dienende Oberfläche Griffflächen oder Griffnoppen bildende erhabene Strukturen aus einem Kunststoffmaterial aufweist. Bei der Herstellung eines solchen Stiftes wird eine wenigstens einen Kunststoff enthaltende Zubereitung in einer zunächst fließfähigen, sich später zu den erhabenen Strukturen verfestigenden Form auf die Oberfläche des Stiftes aufgebracht. Der Hauptbestandteil der Kunststoffmasse ist eine wässrige, wasserfest erhärtende Polymerdispersion oder eine Mischung solcher Dispersionen. Die auf die Stiftoberfläche aufgebrachte wässrige Polymerdispersion kann einen strahlenhärtbaren Kunststoff oder einen physikalischen härtbaren Kunststoffenthalten.

DE 29 38 292 A1 beschreibt ein Verfahren zur Herstellung von Fußbodenbelägen. Im Rahmen eines solchen Verfahrens wird zur Herstellung eines dekorativen Substrates eine noppentexturierte, strahlungsgehärtete Trittschicht geschaffen. Das Herstellungsverfahren verwendet keine Prägung.

Aus DE 26 10 247 A1 geht die Herstellung dünner Gegenstände, wie beispielsweise Deckeln für Fotodosen hervor. Diese sind aus einem vergleichsweise dünnen Formteil aus plastischem Material hergestellt. Die Formteile sind nicht nur extrem dünn, sondern auch vergleichsweise kompliziert geformt, und daher schwierig herzustellen. Zu diesem Zweck werden die Formteile auf einem bandförmigen Träger aufgebracht, an dem sie lediglich so fest haften sollen, dass sie später wieder abgeschabt werden können.

Aus EP 0 181 528 A geht die Herstellung von abbriebfesten UV-härtbaren Beschichtungen hervor. Eine solche Beschichtung soll möglichst fest an Oberflächen von Kunststoffen mit basischen Eigenschaften, wie beispielsweise Polycarbonat und PVC-Harzen haften.

Aufgabe der Erfindung ist es, ein alternatives Verfahren zur Herstellung eines holzgefassten Stiftes mit erhabenen Strukturen aus einem alternativen Material vorzuschlagen.

Die Aufgabe wird gelöst durch ein Verfahren nach Anspruch 1. Demgemäß wird zur Herstellung eines holzgefassten Stiftes für Schreib-, Mal-, Zeichen- und Kosmetikzwecke, eine zunächst fließfähige sich später verfestigende, wenigstens einen Kunststoff enthaltende Zubereitung auf die zur Handhabung des Stiftes dienende Oberfläche aufgebracht. Die aufgebrachte Zubereitung verfestigt sich zu aus der Oberfläche vorstehenden erhabenen Strukturen als Griffflächen oder Griffnoppen. Bei der Zubereitung handelt es sich um eine wasserfreie Zubereitung, die einen strahlungshärtbaren Kunststoff enthält und folgende Zusammensetzung (in Gew.%) aufweist:

| | |
|---|---|
| UV-härtbarer Kunststoff | 40 - 98 % |
| Organisches Lösungsmittel | 0 - 40 % |
| Photoinitiator | 0,1 - 30 % |
| Farbmittel | 0 - 60 % |
| Füllstoffe | 0 - 60 % |

Ein wesentlicher Vorteil des Verfahrens liegt in der Wasserfreiheit der Zubereitung begründet, die eine Vereinfachung der Herstellung mit sich bringt. Bei der Herstellung der aus der EP 1 177 108 B1 bekannten Stifte müssen diese nach dem Auftragen der wässrigen Polymerdispersion bis zu zwei Tage in Trockenräumen gelagert werden. Dementsprechend sind, um die laufende Produktion zweier Tage aufnehmen zu können, große Trockenräume erforderlich, was mit hohen Betriebskosten verbunden ist. Die lange Trockenzeit ergibt sich aus der langsamen Verdunstungsgeschwindigkeit des Wassers der wässrigen Kunststoffdispersionen. Diese kann auch durch Temperaturerhöhung nicht wesentlich verkürzt werden, da die Holzummantelung der Stifte und oft die darin enthaltene Mine dies nicht verträgt. Die Stiftumhüllung kann sich durch eine zu schnelle Wasserabgabe aus dem Holz verziehen oder verwinden. Holzummantelungen sind bekanntlich aus zwei längsverleimten Hälften hergestellt. Bei Anwendung hoher Temperaturen, beispielsweise wie bei JP-09/039467 A angeben, besteht die Gefahr, das sich die Verleimung löst. Bei einem Verfahren nach Anspruch 1 ist dagegen überhaupt kein separater Trockenraum mehr erforderlich. Die Stifte können nach dem Aufbringen der Kunststoffzubereitung an eine UV-Belichtungsstation übergeben werden, in der innerhalb von wenigen Sekunden eine Verfestigung der erhabenen Strukturen auf der Holzummantelung des Stiftes erfolgt. Außerdem ist bei einer lösungsmittelbasierten Kunststoffzubereitung die Trockenzeit wegen des höheren Dampfdruckes organischer Lösemittel gegenüber Wasser wesentlich verkürzt, so dass hier zur Trocknung der Stifte nach dem Aufbringen der Zubereitung Trockenräume mit geringerer Raumkapazität ausreichend sind.

Ein weiteres Problem bei der Herstellung von Stiften der in Rede stehenden Art ist die Handhabung der fließfähigen Kunststoffzubereitung bis zum Auftrag auf die Stiftoberfläche. Bei wässrigen Kunststoffdispersionen besteht die Gefahr, dass es durch Verdunstung von Wasser zu einer Verdickung der Zubereitung und damit zur Ablagerung eines Kunststofffilmes kommt. Wenn sich ein solcher Film im Bereich einer Düse oder beim Auftrag mit Hilfe des Siebdruckverfahrens am Sieb bildet, ist eine aufwändige Reinigung erforderlich, die einen Stillstand der Produktionsanlage bedingt. Besonders nachteilig ist dabei, dass die Polymerfilme mit Wasser nicht mehr auflösbar sind. Dies ist bei lösungsmittelbasierten Zubereitungen anders. Falls hier an Werkzeugen und Vorrichtungen Ablagerungen entstehen, können diese mit dem Lösemittel auf einfache Weise wieder abgelöst werden. Außerdem sind bei Zubereitungen gem. Anspruch 1, bei denen die Filmbildung aufgrund einer UV-Härtung erfolgt, sind organische Lösungsmittel überhaupt nicht oder allenfalls in geringer Menge erforderlich, so dass die Tendenz zur Bildung von verfestigten Ablagerungen reduziert oder gar nicht mehr vorhanden ist.

Schließlich ist noch vorteilhaft, dass aufgrund der Wasserfreiheit eine Korrosion von Vorrichtungsbestandteilen, die mit einer Zubereitung an Berührung kommen, nicht zu befürchten ist.

Bei einer Zubereitung auf der Basis eines UV-härtbaren Kunststoffs ist es vorteilhaft, wenn die Zubereitung lösungsmittelfrei ist. Eine Erhöhung der Viskosität der Zubereitung während des Herstellungsverfahrens durch Verdunstung von Lösungsmittel ist dadurch ausgeschlossen. Eine Veränderung der Viskosität der Zubereitung während des Herstellungsprozesses kann dazu führen, das sich die Form der erzeugten erhabenen Strukturen mit zunehmender Viskosität der Zubereitung verändert. Vorteilhaft ist weiterhin, dass vor der UV-Härtung keine Trocknung erfolgen muss, um noch in den erhabenen Strukturen vorhandenes Lösungsmittel zu entfernen.

Eine gut geeignete UV-härtbare Zubereitung enthält wenigstens ein Acrylat-Derivat, wobei es sich hier um ein Monomer oder ein Oligomer oder um eine Mischung davon handeln kann. Als Oligomere werden vorteilhaft aromatische und aliphatische Epoxy-Acrylate, Polyester-Polyurethan-, Oligoether- und Amin-modifizierte Oligoether-Acrylate verwendet.

Die Zubereitung kann einen Füllstoff enthalten, der vorzugsweise aus der Gruppe Kaolin, Talkum, Bariumsulfat, Titanweiß, Kalciumcarbonat und Glimmer ausgewählt ist. Durch Zusatz eines solchen Füllstoffes kann die Konsistenz der erhabenen Strukturen, die sich natürlich auch aus der Art des verwendeten Kunststoffes ergibt, in weiten Bereichen variiert werden. Zur Beeinflussung der taktilen Eigenschaften der Oberflächen der erhabenen Strukturen eignen sich in besonderer Weise Füllstoffe aus der Gruppe Aluminiumsilikat-Hohlkugeln, expandierte Hohlkugeln, Polyurethan-Soft feeling-Kügelchen, mikronisierte Kunststoffe wie Polypropylen oder PTFE-, und PA-Wachse.

Die Erfindung wird nun anhand eines in der beigefügten Zeichnung dargestellten Ausführungsbeispiels sowie anhand von Zusammensetzungsbeispielen für die Kunststoffmasse näher erläutert.

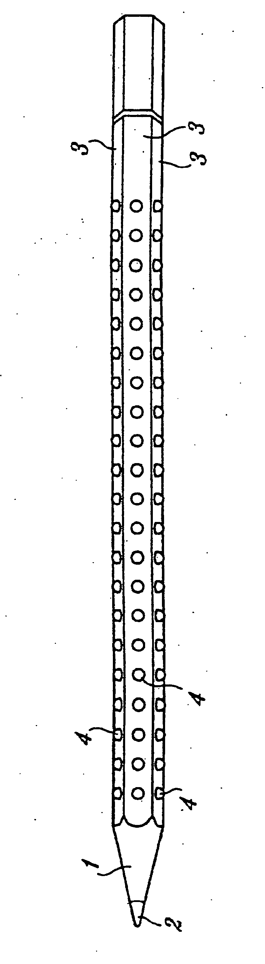

Fig. 1 zeigt einen Bleistift mit einer Holzummantelung 1 und einer Bleistiftmine 2. Der Stift weist eine hexagonale Umrissform auf. Von den einzelnen Hexagonflächen 3 stehen Noppen 4 im Wesentlichen in Radialrichtung hervor. Anstelle von Noppen können auch andere Strukturen auf die Stiftoberfläche aufgebracht sein, beispielsweise sich in Stiftlängsrichtung erstreckende leistenförmige, punktförmige oder beliebige andere Strukturen. Denkbar ist es auch, dass die erhabene Struktur sich ringförmig um den Stiftumfang herum erstreckt. Die erhabenen Strukturen bzw. Noppen können sowohl auf eine blanke bzw. ungefärbte Holzoberfläche als auch auf eine mit einem Lacküberzug versehene aufgebracht sein.
Im Folgenden werden nun einige beispielhafte Rezepturen für Kunststoffzubereitungen angegeben. Die Prozentangaben beziehen sich auf die Kunststoffmasse im fließfähigen, also noch nicht erhärteten Ausgangszustand. Alle Prozentangaben sind Gewichtsprozent, soweit nichts anderes angegeben ist.

### Beispiel:

UV-vernetzbares System für transparente oder farblose Oberflächenstukturen für holzgefasste Stifte.

| | |
|---|---|
| Acrylat-Oligomer (Ebecryl 600/35 OT) | 76,5 % |
| Acrylat-Monomer (Ebecryl 40) | 8 % |
| Benzophenon (Photoinitiator) | 4 % |
| Reaktives tertiäres Amin (Co-Initiator) | 8 % |
| Hydroxy-cyclohexyl-phenylketon(Co-Initiator) | 1 % |
| Verlaufs- und Gleitmittel | 1,5 % |
| Entschäumer | 1 % |

Die UV-härtbaren Acrylate Ebecryl 600/35 OT und Ebecryl 40 sind von der Firma UCB Chemie GmbH, 50170 Kerpen, DE erhältlich. Das eingesetzte tertiäre Amin wird beispielsweise von CIBA Spezialitätenchemie, 68632 Lampertheim, DE hergestellt. Nach der Applikation von Strukturen auf holzgefasste Stifte erfolgt zur Vernetzung eine Bestrahlung mit UV-Licht. Als Verlaufs- und Gleitmittel wird TEGO Glide 440 und als Entschäumer

TEGO Airex 900 eingesetzt, beides Produkte der TEGO Chemie Service GmbH, 45139 Essen, DE.

Die Applikation der Zubereitung auf einen holzummantelten Stift erfolgt im Siebdruck. Kurz nach dem Auftrag der Zubereitung wird der Stift einer UV-Bestrahlungs-Station zugeführt, wo nach UV-Bestrahlung praktisch eine sofortige Härtung eintritt. Neben UV kommt auch eine Bestrahlung mit Elektronen in Betracht. Ein Photoinitiator ist dabei nicht erforderlich.

## Patentansprüche

1. Verfahren zur Herstellung eines holzgefassten Stiftes für Schreib-, Mal-, Zeichen- und Kosmetikzwecke, von dessen der Handhabung dienenden Oberfläche Griffflächen oder Griffnoppen bildende erhabene Strukturen aus einem Kunststoffmaterial vorstehen, die in Form einer zunächst fließfähigen, sich später zu den erhabenen Strukturen verfestigenden, wenigstens einen Kunststoff enthaltenden Zubereitung aufgebracht werden,
**gekennzeichnet durch**
die Verwendung einer wasserfreien Zubereitung, die einen strahlungshärtbaren Kunststoff enthält und folgende Zusammensetzung (in Gew.%) aufweist:
| | |
|---|---|
| UV-härtbarer Kunststoff | 40 - 98 % |
| Organisches Lösungsmittel | 0 - 40 % |
| Photoinitiator | 0,1 - 30 % |
| Farbmittel | 0 - 60 % |
| Füllstoffe | 0 - 60 % |
| Additive | 0 - 10 % |

2. Verfahren nach Anspruch 1
**dadurch gekennzeichnet,**
**dass** die Zubereitung als Photoinitiator Benzophenon und/oder ein Benzophenon-Derivat enthält.

3. Verfahren nach Anspruch 1 oder 2,
**dadurch gekennzeichnet,**
**dass** in der Zubereitung ein die Wirkung des Photoinitiators verstärkender Co-Initiator vorhanden ist.

4. Verfahren nach einem der vorhergehenden Ansprüche,
**dadurch gekennzeichnet,**
**dass** die Zubereitung ein Acrylat-Monomer und/oder ein Acrylat-Oligomer enthält, wobei letzteres ausgewählt ist aus der Gruppe aromatische und aliphatische Epoxidharze, Polyester-, Polyurethan-, Oligoether- , Amin-modifizierte Oligoether und Polyol-Acrylate.

5. Verfahren nach Anspruch 4,
**gekennzeichnet durch**
folgende Zusammensetzung (Gew. %):
| | |
|---|---|
| Acrylat- Oligomer - | 70 - 80 % |
| Acrylat- Monomer | 4 - 12 % |
| Benzophenon | 2 - 8 % |
| Co-Initiator | 5 - 12 % |
| Verlaufs- und Gleitmittel | 1 - 2 % |
| Entschäumer | 0,5 - 1,5 % |

## Claims

1. Process for producing a pencil encased in wood for writing, colouring, drawing and cosmetic purposes, from whose surface, which serves for handling, raised structures, forming grip areas or grip nubs, made of a plastic material protrude, said structures being applied in the form of an initially flowable preparation which later solidifies to give the raised structures and comprises at least one plastic,
**characterized by**
the use of an anhydrous preparation which comprises a radiation-curable plastic and has the following composition (in % by wt.):
| | |
|---|---|
| UV-curable plastic | 40 - 98% |
| organic solvent | 0 - 40% |
| photoinitiator | 0.1 - 30% |
| colorant | 0 - 60% |
| fillers | 0 - 60% |
| additives | 0 - 10% |

2. Process according to Claim 1,
**characterized in that**
the preparation comprises benzophenone and/or a benzophenone derivative as photoinitiator.

3. Process according to Claim 1 or 2,
**characterized in that**
a co-initiator boosting the effect of the photoinitiator is present in the preparation.

4. Process according to one of the preceding claims,
**characterized in that**
the preparation comprises an acrylate monomer and/or an acrylate oligomer, the latter being selected from the group of aromatic and aliphatic epoxy resins, polyester-, polyurethane-, oligoether-, amine-modified oligoethers and polyol acrylates.

5. Process according to Claim 4,
**characterized by**
the following composition (% by wt.):
| | |
|---|---|
| acrylate oligomer | 70 - 80% |
| acrylate monomer | 4 - 12% |
| benzophenone | 2 - 8% |
| co-initiator | 5 - 12% |
| flow agent and lubricant | 1 - 2% |
| antifoam | 0.5 - 1.5% |

## Revendications

1. Procédé de fabrication d'un crayon à gaine de bois à des fins d'écriture, de coloriage, de dessin et cosmétiques, de la surface duquel servant à la manipulation dépassent des structures en une matière plastique en saillie formant des faces de préhension ou des bossages de préhension, structures qui sont déposées sous la forme d'une préparation contenant au moins une matière plastique susceptible d'abord de s'écouler et se solidifiant ultérieurement en les structures en saillie, **caractérisé par** l'utilisation d'une préparation anhydre qui contient une matière plastique pouvant être durcie par un rayonnement et qui a la composition suivante (en %, en poids) :
| | |
|---|---|
| matière plastique pouvant être durcie par les UV | de 40 à 98 % |
| solvant organique | de 0 à 40 % |
| photoamorceur | de 0,1 à 30 % |
| colorants | de 0 à 60 % |
| charges | de 0 à 60 % |
| additifs | de 0 à 10 % |

2. Procédé suivant la revendication 1,
**caractérisé en ce que** la préparation contient comme photoamorceur de la benzophénone et/ou un dérivé de la benzophénone.

3. Procédé suivant la revendication 1 ou 2,
**caractérisé en ce qu'**il y a dans la préparation un co-amorceur renforçant l'effet du photo-amorceur.

4. Procédé suivant l'une des revendications précédentes,
**caractérisé en ce que** la préparation contient un monomère d'acrylate et/ou un oligomère d'acrylate, ce dernier étant choisi dans le groupe des résines époxyde aromatiques et aliphatiques, des acrylates de polyester, des acrylates de polyuréthane, des acrylates d'oligoéther, des acrylates d'oligoéther modifiés par une amine et des acrylates de polyol.

5. Procédé suivant la revendication 4,
**caractérisé par** la composition suivante (en %, en poids) :
| | |
|---|---|
| Oligomère d'acrylate | de 70 à 80 % |
| Monomère d'acrylate | de 4 à 12 % |
| Benzophénone | de 2 à 8 % |
| Co-amorceur | de 5 à 12 % |
| Agent nivelant et lubrifiant | de 1 à 2 % |
| Agent anti-mousse | de 0,5 à 1,5 %. |
